# EUROPEAN PATENT APPLICATION

(11) **EP 3 766 474 A1**
(43) Date of publication of application: **20.01.2021**
(21) Application number: 19768495.4
(22) Date of filing: 15.03.2019
(51) Int. Cl.: A61K 8/02, A61K 8/34, A61K 8/92, A61Q 19/00

(54) **MASSAGE CANDLE CONTAINING CANNABIDIOL OR ESSENTIAL OIL**

(30) Priority: 16.03.2018 JP 2018048830
(71) Applicant: Iijima, Noriko, Tokyo 106-0047 (JP)
(72) Inventor: Iijima, Noriko, Tokyo 106-0047 (JP)
(74) Representative: Schön, Christoph
(86) International application number: PCT/JP2019/010895
(87) International publication number: WO 2019/177155

(57) **Abstract**

The present invention addresses the problem of providing a massage oil capable of alleviating pain of the muscles, joints, nerves, etc. In order to solve this problem, there is provided a massage candle that has a container, a wax component accommodated in the container, and a wick inserted into the wax component, wherein the melting point of the wax component is 30-60°C, the wax component contains 40% by weight or more of shea fat and also contains 15-40% by weight of vegetable oil that is liquid at normal temperature, and the massage candle is for alleviating pain.

## Description

### TECHNICAL FIELD

The present invention relates to a candle for massage. More specifically, the present invention relates to a candle for massage containing cannabidiol or an essential oil for relieving pain in muscles, joints, nerves, and the like.

### BACKGROUND OF THE INVENTION

Massage may be performed using oil for massage. The use of the oil for massage makes the finger slippery, so that massage can be effectively performed. For example, Patent Document 1 discloses an oil for massage containing essential oil of millet. The invention in the Patent Document 1 is that it can be continuously used for a person who is sensitive to alcohol or a person who has weak skin by using essential oil of millet extracted from the leaves or species of millet by steam distillation method. by using essential oil of millet extracted from the leaves or species of millet by steam distillation method.
In addition, Patent Document 2 discloses a coconut oil cream obtained by mixing a gamma cyclodextrin inclusion body of beeswax and delta tocotrienol with coconut oil. This document describes the effect of oil massage using coconut oil to relieve pain in muscles, joints, nerves, and the like.
In addition, Patent Document 3 describes a candle for massage, which contains 40 wt.% (% by weight) or more of shea fat as a wax component, contains 15 wt.% or more and 40 wt.% or less of a vegetable oil in a liquid state at ambient temperatures, and has a wax component having a melting point of 30 °C or more and 60 °C or less. According to the invention in the Patent Document 3, the oil for massage is warmed to an appropriate temperature, so that the feeling is comfortable and the effect of the components contained in the oil appears quickly.

### PRIOR ART DOCUMENT

### [Patent Document]

[Patent Document 1] JP-A-2005-194408
[Patent Document 2] JP-A-2017-81839
[Patent Document 3] JP Patent No. 5,087,675

### SUMMARY OF THE INVENTION

### [PROBLEM TO BE SOLVED BY THE INVENTION]

As the effect of oil massage, the effect of relieving the pain of muscle, joint, nerve, and the like is expected. Therefore, an object of the present invention is to provide a massage oil capable of more relieving pain in muscles, joints, nerves, and the like.

### [MEANS FOR SOLVING THE PROBLEMS]

As a result of extensive studies on the above problems, the present inventor has completed the present invention by finding that a candle for massage which contains 40 wt.% or more of shea fat as a wax component, 15 wt.% or more and 40 wt.% or less of a vegetable oil in a liquid state at ambient temperatures, and is provided with a wax component having a melting point of 30 °C or more and 60 °C or less, exhibits a particularly excellent effect with respect to an action of relieving pain.
That is, the present invention is a candle for massage as follows.

A candle for massage according to the present invention for solving the above problem is a candle for massage comprising: a container; a wax component contained in the container; and a wick inserted into the wax component, wherein a melting point of the wax component is 30 °C or more and 60 °C or less, and wherein the wax component contains 40 wt.% or more of shea fat and contains 15 wt.% or more and 40 wt.% or less of a vegetable oil in a liquid state at ambient temperatures.
The candle for massage has an effect superior to other massage oils in relieving pain in muscles, joints, nerves, and the like.
Further, according to the candle for massage, in addition to the above-mentioned effect, the basal body temperature is increased by long-term use, and the effect of improving poor circulation and the like is also recognized.

A candle for massage according to the present invention for solving the above problem is a candle for massage, comprising: a container; a wax component contained in the container; and a wick inserted into the wax component, wherein a melting point of the wax component is 30 °C or more and 60 °C or less, and the wax component includes 40 wt.% of shea fat, 15 wt.% or more and 40 wt.% or less of a vegetable oil in a liquid state at ambient temperatures, and an essential oil.
The candle for massage exhibits a particularly excellent effect in relieving pain in muscles, joints, nerves, and the like.

### [EFFECT OF THE INVENTION]

According to the present invention, a massage oil capable of more relieving pain of muscles, joints, nerves, and the like can be provided.

### BRIEF DESCRIPTION OF THE DRAWINGS

Figure 1 is a conceptual diagram showing a candle for massage of the present invention.
Figure 2 is thermography showing the change of body surface temperature by the treatment using the candle for massage of the present invention.
Figure 3 is thermography showing the change of the body surface temperature by the treatment using the aroma oil of a ready-made product.
Figure 4 is a graph showing changes of body temperature (before treatment) by treatment using a candle for massage of the present invention.
Figure 5 is a graph showing changes of body temperature (from before to 4 hours after treatment) by treatment using a candle for massage of the present invention.

### [FORMS FOR IMPLEMENTING THE INVENTION]

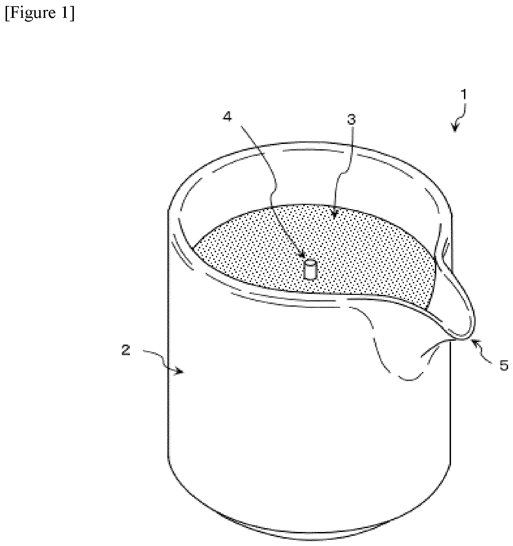

Hereinafter, Embodiments of the present invention will be described below. FIG. 1 is a conceptual diagram showing a candle for massage of the present invention. As shown in FIG. 1, the candle for massage of the present invention has a container 2, a wax component 3 contained in the container 2, and a wick 4 inserted into the wax component 3.

The container shown in FIG. 1 is shaped like a beaker. However, the shape of the container 2 is not limited to a beaker-like one. Preferably, the container 2 has a spout 5 for pouring the dissolved wax component. The spout 5 is usually provided above the container 2. Then, through the spout 5, the liquid in the container can be poured out of the container. Preferably, the container 2 has heat resistance. An example of the container 2 is made of pottery. On the other hand, one made of plastic may be used as the container 2. The size of the container 2 may be appropriately adjusted according to the application. Examples of the volume of the container 2 include 10 ml or more and 1 liter or less, or possibly 50 ml or more and 500 ml or less.

The wax component 3 is the main body of the candle. When a fire is applied to the wick 4, the wax component 3 is melted, and a part of the wax component 3 is burned.

The wax component 3 contains a solid fat and a liquid oil, and contains 40 wt.% or more of shea fat as a solid fat, and contains 15 wt.% or more and 40 wt.% or less of a vegetable oil in a liquid state at ambient temperatures (20 °C) as a liquid oil. A solid fat is a fat having a melting point of 20 °C or higher, and a liquid oil is an oil having a melting point of less than 20 °C.

Shea fat means the fat content and the extracts and contents contained therein from the embroidery of the seed of Shea trees (Butyrospermum Parkii family). Shea fat has butter-like properties. Therefore, shea fat is also called shea butter. Shea fat, which is solid at ambient temperatures, has a low melting point of about 35 to 45 °C. As the shea fat of the present invention, not only shea fat in a narrow sense but also an extract or an oil or other material extracted from a shea tree may be contained.

Further, the wax component 3 may contain a solid fat other than shea fat. Examples of solid fats are vegetable fats, animal-derived fats, waxes, hydrocarbon oils, higher fatty acids, and higher alcohols. The wax component may include one kind of solid fat. Further, the wax component may contain two or more kinds of solid fats.

Examples of vegetable solid fats are shea fat, cocoa butter, mango seed fat, and vegetable waxes. Examples of vegetable waxes are carnauba wax, ouricury wax, palm wax, candelilla wax, sugar cane wax, cotton wax, flax wax, okochira wax, pisang wax, and esparto wax.

Examples of animal-derived fats are beef tallow, sheep tallow, horse fat and lard. Examples of waxes are beeswax, bead wax, in addition to the vegetable waxes described above. Examples of hydrocarbon oils are petrolatum and polyolefins. Examples of higher fatty acids are palmitic acid, stearic acid, myristic acid, behenic acid, lauric acid, myristic acid, eicosapentaenoic acid and docosahexahydrochloric acid. An example of a higher alcohol is octyldodecanol.

The content of the solid fat is 40 wt.% or more, preferably 50 wt.% or more, and further preferably 60 wt.% or more.

Liquid oil means oils and fats which are in a liquid state at ambient temperatures (20 °C). Examples of liquid oils are vegetable oils, animal oils, hydrocarbon oils, higher fatty acids, higher alcohols, silicone oils, esters, and glycerins. The wax component 3 may contain two or more kinds of liquid oils. The wax component 3 preferably contains 15 wt.% or more and 40 wt.% or less of a liquid oil. The lower limit value of the content of the liquid oil is more preferably 20 wt.% or more. The upper limit value is more preferably 35 wt.% or less, and further preferably 30 wt.% or less.

Examples of vegetable oils are palm oil, almond oil, coconut oil, vegetable oil, carapaguaianensis seed oil, avocado oil, camellia oil, turtle oil, macadamia nut oil, corn oil, sesame oil, persic oil, wheat germ oil, sasanqua oil, castor oil, linseed oil, safflower oil, cotton seed oil, perilla oil, soybean oil, peanut oil, tea seed oil, kaya oil, rice bran oil, jojoba oil, apricot kernel oil, olive oil, carrot oil, grapeseed oil, rapeseed oil, camellia oil, and jojoba oil.

Examples of animal oils are egg yolk oil and mink oil. Examples of hydrocarbon oils are liquid paraffin, squalene, and squalane. Examples of higher fatty acids are oleic acid, tall oil, and isostearic acid. Examples of higher alcohols are lauryl alcohol, oleyl alcohol, isostearyl alcohol, and octyldodecanol. Examples of silicone oils are methylpolysiloxane, methylphenylpolysiloxane, methylhydrogenpolysiloxane, and decamethylpolysiloxane. Examples of esters are isopropyl myristate, isopropyl palmitate, hexyl laurate, oleyl oleate, decyl oleate, octyldodecyl myristate, hexyldecyl dimethyl octanoate, diethyl phthalate, and dibutyl phthalate. Examples of glycerins include one or two or more of glycerins such as glycerin, diglycerin, triglycerin, glycerin trioctanoate, and glycerin triisopalmitate.

Among the liquid oils, palm oil is suitable. When the wax component contains palm oil, the oil when the wax component is dissolved becomes smooth and easily extended. As the liquid oil, the liquid oil containing palm oil and vegetable oil other than palm oil is preferably used.

The melting point of the wax component 3 is 30 °C or more and 60 °C or less. The lower limit is preferably 35°C or more. The upper limit is preferably 55 °C or less, more preferably 50 °C or less. By adjusting the type and blending ratio of the solid fat and the liquid oil, the melting point of the wax component 3 can be adjusted. For example, by adding a higher fatty acid having a melting point of 50 °C or more, it is possible to increase the melting point of the wax component.

The wax component 3 may contain other ingredients. Other ingredients include, for example, cannabidiol, thickeners, essential oils, nutritional ingredients, and the like.

Cannabidiol is a compound shown in the following chemical formula (1). By adding cannabidiol, the effect of the present invention of relieving pain can be further exhibited. [Chemical formula (1)]

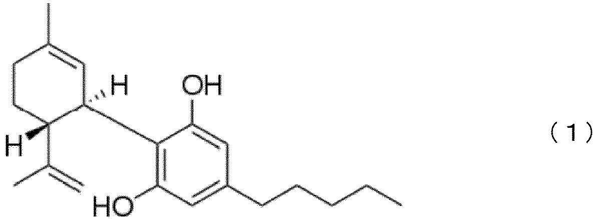

The content of cannabidiol in the wax component is not particularly limited, but is preferably 0.001 to 30 wt.%. As the lower limit, more preferably 0.01 wt.% or more, further preferably 0.1 wt.% or more, and particularly preferably 0.3 wt.% or more. As the upper limit, more preferably 10 wt.% or less, further preferably 5 wt.% or less, and particularly preferably 3 wt.% or less.

The wax component 3 preferably contains 2 wt.% or more and 15 wt.% or less of a thickener. When the thickener is added to the wax component 3, the composition can be effectively produced by using the solid fat and the liquid oil. An example of a thickener is silicic anhydride. As the silicic anhydride, silylated silicic anhydride is preferred.

The wax component 3 may further contain essential oils. Essential oils are volatile oils obtained from plants. By adding essential oils, the candle for massage of the present invention also exhibits an aromatherapy effect. As the essential oil, known materials already known in the art can be appropriately used. Examples of essential oils are Angelica, Benzoin, Oregano, Orange, Chamomile, Kayupte, Galvanum, Clarisage, Grapefruit, Grapefruit peel, Cypress (Italian cypress), Sandalwood, Ceder wood, Citronella, Cinnamon, Jasmine, Juniper berry, Ginger, Spike lavender, Spearmint, Sage, Geranium, Thyme, Tangerine, Tea tree, Nutmeg, Frankincense, Neroli, Pine, Basil, Patchouli, Verbena, Rose, Palmarosa, Bitter orange flower, Fennel, Petitgrain, Vetiver, Peppermint, Bergamot, marjoram, mandarin orange peel, melissa leaves (Lemon balm), eucalyptus, lavandin, lavender, lemon, lemongrass, rosewood, rosemary, and Roman chamomile and the like. The wax component may contain only one type of essential oil. Further, the wax component may contain two or more kinds of essential oils. Examples of the content of the essential oil are 0.001 wt.% or more and 10 wt.% or less, preferably 0.003 wt.% or more and 5 wt.% or less, and particularly preferably 0.1 wt.% or more and 3 wt.% or less.

The wax component may contain nutritional ingredients to the skin. Examples of nutritional ingredients include trehalose and vitamins. Since trehalose has a moisturizing effect and a damage recovery effect, it is preferable that the wax component contains trehalose.

As the wick 4, a normal wick used for candles can be used. Preferably, the wick 4 has a portion which is inserted into the wax component 3 and a portion which protrudes from the wax component 3. By applying a fire to the tip of the wick 4 protruding from the wax component 3, heat can be transferred to the wax component and the wax component can be melted. The wick 4 may be rod-shaped. When the wick 4 is rod-shaped, the diameter of the wick 4 is 1 mm or more and 3 mm or less, possibly 1.5 mm or more and 2.5 mm or less.

By modifying and using a method for producing a candle already known, the candle for massage of the present invention can be produced. For example, first, the solid fat is heated to a liquid. Then, liquid oil and other ingredients are added and stirred. Thus, a composition constituting a wax component can be obtained. The composition for the wax component is filled into a container while remaining in a liquid state. Thereafter, the wick is set. When the wax component of the liquid is cooled, the wax component solidifies. In this way, the candle for massage of the present invention can be produced.

A second aspect of the present invention relates to a method of obtaining an oil for massage. This method is basically a method of obtaining the oil using the candle for massage of the present invention. The method includes a step of providing the candle for massage, a step of igniting the wick, and a step of liquefying the wax component by the fire applied to the wick. This makes it possible to obtain the oil for massage at an appropriate temperature. In the step of liquefying the wax component, the liquefaction of the wax component means a state in which the wax component in an amount that can be used for massage is liquefied. The time for the wax component to liquefy varies with the composition of the wax component. Examples of the time for liquefying the wax component is one second or more and one minute or less, and preferably five seconds or more and thirty seconds or less.

Upon ignition of the wick, the heat of the flame melts the wax component below the wick. Because the candle for massage of the present invention has a lower melting point than a conventional candle, melted oil accumulates at the top of the wax component.

As a method of obtaining a liquefied oil, a method of dissolving a wax using a hot water helix (hot water bath) is conceivable. However, when the wax is indirectly heated by using a hot water bath, the oil obtained by liquefying the wax is easily cooled. Therefore, in the case of using a hot water bath, the appropriate temperature cannot be maintained for a long time, and therefore, the treatment effect cannot be sufficiently obtained.

On the other hand, in the method using the candle of the present invention, the wick is applied a fire to make the candle liquid. The oil obtained for this purpose can be maintained at a temperature of 38 °C or more and 42 °C or less, which is suitable for massage, for a long time. This method using candles does not consume more candles than necessary because the candles are oiled to an amount just optimal for massage. Further, since the temperature of the oil is an appropriate temperature, a situation in which the essential oil volatilizes can be prevented to a minimum. Also, since the portion to be heated is not the entire candle, deterioration of the component due to warming (e.g., oxidation) can be minimized. In the appropriate temperature state, the oil smoothly expands, which makes it easier for the practitioner to perform the treatment, thereby improving the treatment efficiency. Thus, these active ingredients can be rapidly absorbed into the blood, and the active ingredient can be effectively utilized. In addition, comfort due to a pleasant temperature can be given to the person who is treated the massage, and the massage effect can be enhanced by quickly and effectively exhibiting the performance of the active ingredient.

The third aspect of the present invention relates to a massage method for beauty using the oil for massage of the present invention. This method includes a step of liquefying a candle for massage of the present invention to obtain a liquid oil; and a step of performing massage using liquid oil.

Since this massage method uses a candle for massage of a novel form, an expectation sense and a sense of luxury are enhanced. In addition, the flame provides a visual effect that makes the person who is treated the massage more relaxed. If the wax components contain perfumes, the effect of aromatherapy can also be given.

In the step of performing the massage, the oil for massage may be directly poured on the skin of the person to be treated. Massage oil may also be poured in the practitioner's hand and used. The temperature of the oil is basically the melting point of the wax component. Before the wax component is all melted, they do not go above the melting point. In the step of performing the massage, the oil for massage may be directly poured on the skin of the person to be treated. The oil for massage may also be poured in the practitioner's hand and used. The temperature of the oil is basically the melting point of the wax component. Before the wax component are all melted, they do not go above the melting point. The oil for massage is preferably at a temperature that does not burn and that the massager feels warm.
The specific temperature of the massage oil is 35 °C or more and 45 °C or less, and preferably 37 °C or more and 42 °C or less.

The step of performing massage preferably includes a step of performing massage along the Langer line. The Langer line is a trace of the cell membrane produced by the process of cell division. The running of the Langer Line (the direction of the layer that forms the Langer Line) is also similar to the running of the fibrous (collagen fibers) in the reticular layer of the dermis of the skin. Therefore, massage along the Langer line can prevent a situation in which an excessive force is applied to the skin, so that it is difficult to cause wrinkles of the skin (epidermis and dermis), and it is possible to promote the circulatory system such as blood vessels and lymphatic vessels densely clustered in the reticular layer. Also, the rapid prevalence of essential oils masked by warm oils throughout the body can provide a very high massage effect.

### [Examples]

### [Preparation of candles for massage]

Table 1 shows the composition in Examples and Comparative Examples. As a comparative example, conventional candles (high melting point), and those having a low melting point were also produced. As silicic anhydride, those subjected to silylation treatment were used.

The shea fat and the liquid oil or solid fat (oil or fat) other than palm oil used in each of the examples are as follows.
Example 2: Coconut oil mixed with aloe vera leaf extract
Example 3: Mango seed fat
Example 4: Hydrogenated soybean oil
Example 5: A mixture of almond oil and hydrogenated vegetable oil
Example 6: Carapa Guaianensis Seed Oil
Example 7: Cocoa butter

Note that the term "oil" described above also includes a solid fat. Various perfumes were added to these Examples and Comparative Examples. The amount of perfume added was several hundred ppm.

### [Table 1]

**Table 1 Composition ratio of wax component in Examples and Comparative Examples**

| | Examples | | | | | | | | Comparative Examples | |
|---|---|---|---|---|---|---|---|---|---|---|
| | 1 | 2 | 3 | 4 | 5 | 6 | 7 | 8 | 1 | 2 |
| Shea fat | 60 | 58 | 55 | 52 | 48 | 60 | 56 | 80 | - | 40 |
| Oil or fat | - | 20 | 25 | 25 | 30 | 5 | 32 | - | - | - |
| Palm oil | 30 | 10 | 11 | 12 | 7 | 12 | 5 | - | - | 60 |
| Silicic anhydride | 5 | 5 | 7 | 4 | 7 | 11 | 4 | 5 | - | - |
| Palmitic acid | 5 | 5 | 1 | 4 | 4 | 7 | 2 | 10 | - | - |
| Stearic acid | - | 2 | 1 | 3 | 4 | 5 | 1 | 5 | - | - |
| Commercially available candle | - | - | - | - | - | - | - | - | 100 | - |
| Melting point(°C) | 40 | 42 | 41 | 45 | 48 | 51 | 40 | 58 | 82 | 25 |

In Table 1, a numerical value other than the melting point means wt.%. Note that, as Comparative Example 1, a commercially available candle was used. The candle does not contain shea fat. The candle of Comparative Example 1 had a melting point of 82 °C. Using the candle of Comparative Example 1, it was likely to burn hot when touched the skin. It is considered that the candle of Comparative Example 1 cannot be used for massage.

Comparative Example 2 did not solidify because the melting point of the mixture was low. Therefore, it was not possible to obtain a wax component even when the composition of Comparative Example 2 was used.

The candles for massage of Examples 1 to 7 had a suitable melting point. Obtaining massage oil from this candle and applying the oil to the skin resulted in good oil extension and easy massage.

On the other hand, the candle for massage of Example 8 could be used for massage, although the extension of the oil was not good.

### [Test of pain relief effects]

Next, the candle for massage of Example 1 and the massage oil of Comparative Example 2 were used to perform massage on the affected area of the pain, and the effect of relieving the pain was evaluated. In the candle for massage of Example 1, a melted liquid content (about 40 °C) was used by igniting the wick. The massage oil of Comparative Example 2 was used at ambient temperatures. The evaluation was conducted by applying the candle for massage of Example 1 to the right leg and the massage oil of Comparative Example 2 to the left leg of 5 subjects with muscular pain in the calf of both legs, and asking whether the muscular pain of the left and right legs was relieved in each subject. As a result, it was answered that the muscle pain was relieved in the right leg (Example 1) compared with the left leg (Comparative Example 2) in all five patients. From this result, it has been found that by using the candle for massage of the present invention, it is excellent in the effect of relieving pain.

Next, a candle for massage (Example 9) in which 0.5 wt.% of cannabidiol was added to the candle for massage of Example 1, and a candle for massage (Example 10) in which 2 wt.% of essential oil was added to the candle for massage of Example 1 were prepared. These formulations are shown in Table 2 below.

### [Table 2]

**Table 2 Composition of candle for massage of Examples Unit: wt%**

| | | Examples | |
|---|---|---|---|
| | | 9 | 10 |
| Candle for massage of Example 1 | | 99.5 | 98 |
| Cannabidiol | | 0.5 | - |
| Essential oils | Lavender oil | - | 2 |
| | Geranium oil | | |
| | Rosewood oil | | |
| | Grapefruit extract | | |
| | Rosemary oil | | |
| | Roman chamomile oil | | |

Next, the candle for massage of Example 1 and the candle for massage of Example 9 were evaluated in the same way by comparison for the effect of relieving pain. Both candles for massage were applied a fire on the wick and dissolved liquid content (approximately 40 °C) was used. Five subjects applied the candle for massage of Example 1 to the right leg and the candle for massage of Example 9 to the left leg. As a result, 4 out of 5 responded that the muscle pain was relieved in the left leg (Example 9) than in the right leg (Example 1).
From this result, it was confirmed that the effect of relieving the pain was further improved by adding cannabidiol.

Further, the candle for massage of Example 1 and the candle for massage of Example 10 were compared and evaluated for the effect of relieving pain in the same ways. Both candles for massage were applied a fire on the wick and dissolved liquid content (approximately 40 °C) was used. Five subjects applied the candle for massage of Example 1 to the right leg and the candle for massage of Example 10 to the left leg. As a result, 4 out of 5 responded that the muscle pain was relieved in the left leg (Example 10) than in the right leg (Example 1).
From this result, it was confirmed that the effect of relieving the pain was further improved by adding the essential oil.

### [Test on Changes in Basal Body Temperature]

Next, massage was performed using a candle for massage of Example 9 and an aroma oil of a commercial product, and a change in the base body temperature was evaluated. The evaluation method was as follows. A massage using a candle for massage of Example 9 and a massage using a ready-made aroma oil were applied to one subject for each for 4 consecutive weeks of massage once a week. The candle for massage of Example 9 was applied a fire on a wick and a dissolved liquid content (about 40 °C) was used. The ready-made aroma oil was used at a temperature of about 40 °C. In the massage using the candle for massage of Example 9, a step of performing massage along the Ranger line was performed for 30 minutes, and then, a normal massage was performed for 30 minutes. In the massage using the ready-made aroma oil, the normal massage was performed for 60 minutes (2 times the same process as the normal massage performed with the candle for massage of Example 1).

### (Change in body surface temperature by thermography)

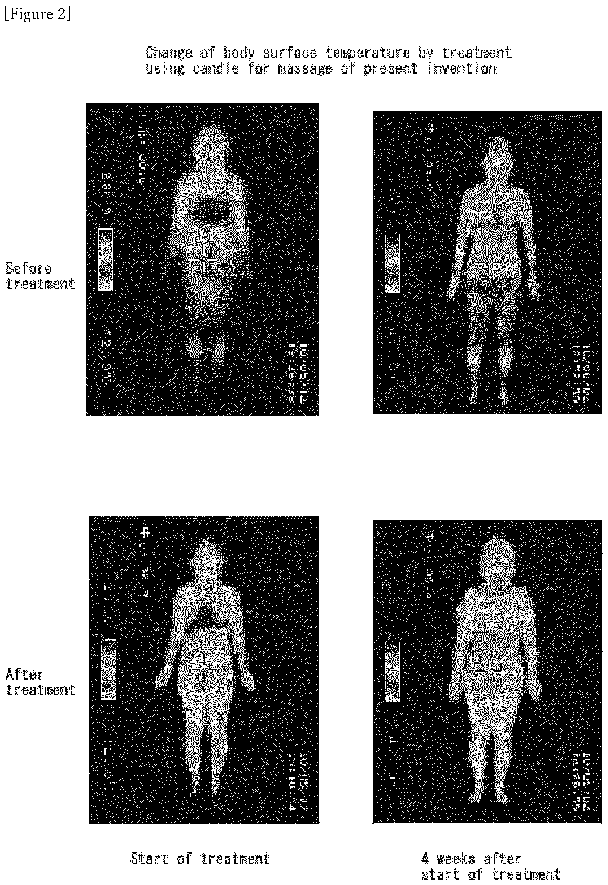

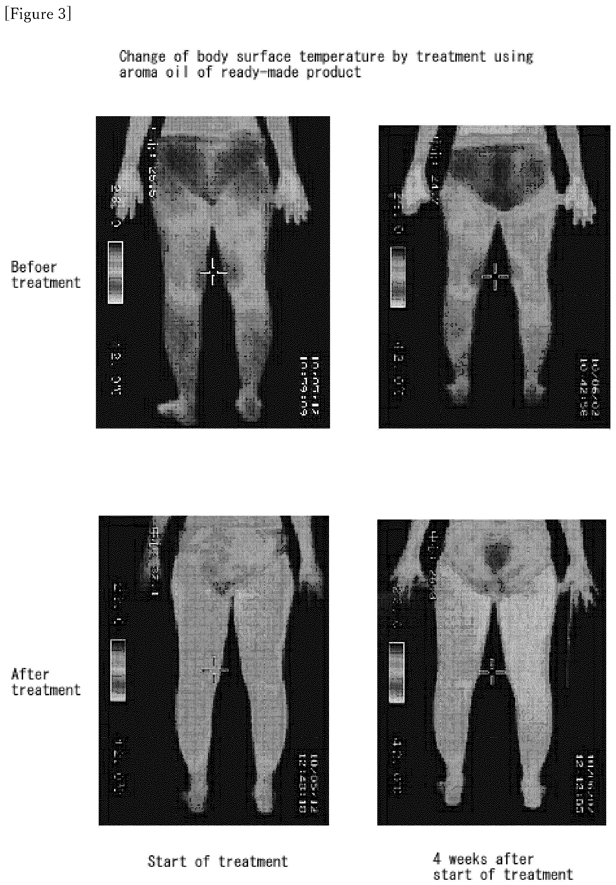

The body surface temperature was confirmed by thermography before and immediately after massage treatment in each week. FIG. 2 shows a change in body surface temperature due to the treatment using the candle for massage of the present invention. FIG. 3 shows a change in body surface temperature due to the treatment using the aroma oil of the off-the-shelf product. FIG. 2 and 3 show thermography before massage treatment (upper left) and after massage treatment (lower left) at the start of treatment (Week 1), and before massage treatment (upper right) and after massage treatment (lower right) at 4 weeks after the start of treatment.

Looking at FIG. 2, it can be seen that the body surface temperature is increased both before and after the massage of the present invention in 4 weeks after the massage using the candle for massage of the present invention, as compared with the time when the massage is started. From these results, it is inferred that the body's blood flow was improved and poor circulation was improved by the massage using the candle for massage of the present invention. Furthermore, it can be said that the constitution is changed to the constitution that the toxin in the body is easily excreted by the improvement of the blood flow.

On the other hand, as shown in FIG. 3, even at the fourth week after the massage using the aroma oil of the ready-made product was performed, the body surface temperature was increased both before and after the operation as compared with the time of the start of the operation. However, the effect was small compared with the massage using the candle for massage of the present invention.

### (Change in basal body temperature due to treatment)

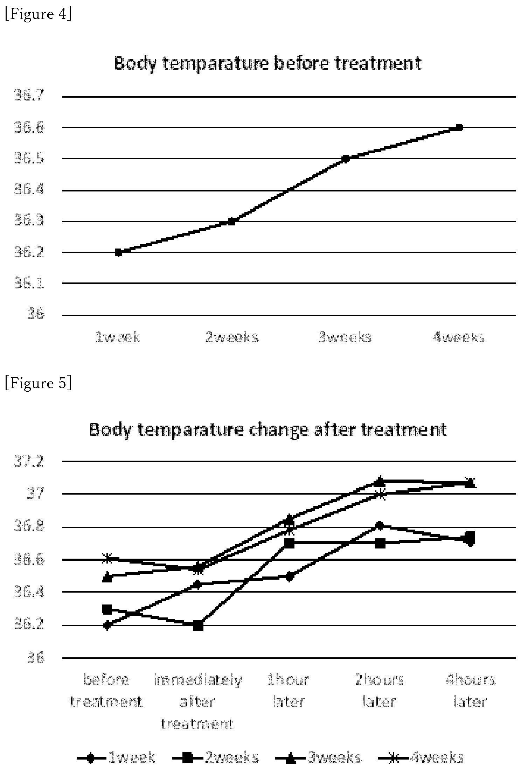

Before and after the massage using the candle for massage of the present invention, body temperature was measured using the basal thermometer. The body temperature was measured immediately after, 1 hour after, 2 hours after, and 4 hours after the treatment. FIG. 4 is a graph showing changes in body temperature before the treatment by treatment using the candle for massage of the present invention. From this graph, it can be seen that the basal body temperature of the whole body before the operation gradually rises each time the massage using the candle for massage of the present invention is repeated. From this result, it is inferred that poor circulation can be improved. FIG. 5 is a graph showing changes in basal body temperature (before and 4 hours after the operation) by the operation using the candle for massage of the present invention. From this graph, it can be seen that, after receiving a massage using a candle for massage of the present invention, the body temperature is increased even after the lapse of time. From this result, it can be understood that blood flow is improved after the massage, and a beautiful skin effect and a lean body effect are expected.

### [Industrial Applicability]

The present invention can be suitably used in the cosmetic industry. It can also be used as a massage candle for relieving muscle and joint pain caused by sports, labor, and the like.

It can also be used for a massage candle for improving blood flow, a massage candle for improving poor circulation, a massage candle for improving skin quality, a massage candle for promoting a lean body, a massage candle for promoting detoxification, and the like.

### [Description of Symbols]

1 Candle for Massage
2 Container
3 Wax Component
4 Wick
5 Spout

## Claims

1. A candle for massage, comprising:
a container;
a wax component contained in the container; and
a wick inserted into the wax component,
wherein a melting point of the wax component is 30 °C or more and 60 °C or less, and
the wax component includes 40 wt.% of shea fat, 15 wt.% or more and 40 wt.% or less of a vegetable oil in a liquid state at ambient temperatures, and cannabidiol.

2. A candle for massage, comprising:
a container;
a wax component contained in the container; and
a wick inserted into the wax component,
wherein a melting point of the wax component is 30 °C or more and 60 °C or less, and
the wax component includes 40 wt.% of shea fat, 15 wt.% or more and 40 wt.% or less of a vegetable oil in a liquid state at ambient temperatures, and an essential oil.

3. The candle for massage according to claim 1 or 2, wherein the candle for massage is used to relieve pain.
